# EUROPEAN PATENT SPECIFICATION

(11) **EP 4 340 738 B1**
(45) Date of publication and mention of the grant of the patent: **10.09.2025**
(21) Application number: 22730133.0
(22) Date of filing: 17.05.2022
(51) Int. Cl.: A61B 10/00, B01L 3/00

(54) **SAMPLING DEVICE**
PROBENAHMEVORRICHTUNG
DISPOSITIF D'ÉCHANTILLONNAGE

(30) Priority: 21.05.2021 GB 202107301
(43) Date of publication of application: 27.03.2024
(73) Proprietor: Readygo Diagnostics Limited, North Wraxall Chippenham Wiltshire SN14 8RY (GB)
(72) Inventor: COBB, Benjamin David, North Wraxall Wiltshire SN14 8RY (GB)
(74) Representative: Marks & Clerk LLP
(86) International application number: PCT/EP2022/063335
(87) International publication number: WO 2022/243323

(56) References cited:
- WO-A1-2012/145379
- WO-A1-2017/142571
- WO-A1-2017/160838
- WO-A2-2005/082254
- US-A- 4 353 868
- US-A- 5 266 266

## Description

### FIELD OF THE INVENTION

The present invention relates to collecting clinical samples and preparing them for analysis, for example by molecular biology processing techniques such as nucleic acid amplification and/or detection; and more particularly to a sampling device for that purpose, especially useful in a clinical point of care (POC) or point of need (PON) setting, but also for use in sending to a remote laboratory for testing. It further envisages the device in the form of a multifunctional kit from which a purpose-specific embodiment can be assembled, suited to the investigation under consideration.

### BACKGROUND TO THE INVENTION

The Polymerase Chain Reaction (PCR) is a convenient method of amplifying nucleic acids, employed to test for the presence of specific nucleic acids (DNA or RNA) in a biological sample. It is used among other purposes as a diagnostic method to identify markers for pathogens or diseases. Other methods of amplifying nucleic acid samples include isothermal amplification methods such as Recombinase Polymerase Amplification (RPA) and Loop-Mediated Isothermal Amplification (LAMP).

Prior to any such diagnostic test there needs to be a certain amount of preparation of the sample in order to present nucleic acids in a state that is compatible with the amplification process being used. Laboratory-based extraction methods are generally geared towards providing high concentrations of high purity nucleic acid, the aim being to obtain as much nucleic acid as possible. This comes at the expense of simplicity, and lab-based extraction techniques often require access to reagents and equipment such as centrifuges that are not compatible with clinical settings.

Preparation of clinical samples requires just enough nucleic acid to perform the test. This is an important distinction because it offers significant simplification of the extraction process. It is known for example, that the use of a simple filter paper enables the capture of enough nucleic acid from a target in a biological sample for a subsequent diagnostic via PCR, even from complex sample matrices such as whole blood (Fuehrer et al. J Clin Microbiol. 2011, 49(4), 1628-1630; Bu et al. Analytical Biochemistry 375(2), 370-372; Zou et al (2017) Nucleic acid purification from plants, animals and microbes in under 30 seconds, PLoS Biol 15(11), e2003916).

International patent application PCT/EP2021/057315, published as WO2021209228A1, in the name of Readygo Diagnostics Limited, describes a sampling device for collection and processing of biological samples, particularly - but not exclusively - for the purpose of DNA and RNA analysis procedures. The contents of that application are incorporated herein by reference. The device described therein comprises:
(a) a nib having a working surface exposed or exposable for acquiring a biological sample, and having also a porous structure suitable for the absorption of biological sample matter thus acquired;
(b) a body having a form suitable for holding in, and manipulation by, the hand, and wherein the nib is connected or connectable to the body; and
(c) a reservoir adapted for fluid communication with the nib to provide the passage of fluid through the nib.

The present inventor has now designed a modified version of that device, and has determined that the modified features permit improvements in sample collection and processing.

US 5 266 266 A discloses a sampling device according to the preamble of claim 1.

### SUMMARY OF THE INVENTION

The sampling device described in International patent application PCT/EP2021/057315 includes a porous, absorbent nib retained within a body suitable for holding in and manipulation by the hand, and a reservoir adapted for fluid communication with the nib to provide the passage of fluid through the nib. In use, a biological sample - for example, a sample containing DNA and/or RNA - is absorbed onto the nib. Fluid is then passed from the reservoir through the nib in order to release and wash the sample, and to pass it into a reaction vessel where a suitable assay may be performed. The present invention adapts this design by incorporation of a snap valve located between the reservoir and the nib, so as to prevent passage of fluid until such time as the valve is opened. Although the device is primarily described herein in conjunction with use in molecular diagnostic testing for nucleic acids, it will be appreciated that other biomolecules may be collected and/or processed with the invention, for example, proteins, lipids etc; as may cell fragments such as cell membrane or cell coat fragments, or subcellular organelles.

The aim of the invention is to provide a configuration of the device that enables collection of different sample types which may exist in various physical forms such as liquids (e.g. blood, saliva or urine) or as biofilms or cellular material on the surface of tissues or objects (e.g. touch surface DNA samples in forensics).

Also described are optional modifications to the basic form of the device that enable concentration of components (eg, nucleic acids) from the sample in order to enhance sensitivity, or that enable larger volumes of sample to be obtained, or to be obtained more simply.

According to a first aspect of the present invention there is provided a device for obtaining biological samples for analysis, as defined in claim 1.

The frangible plug (which may also be referred to herein as a snap valve) thus prevents fluid flow until it is opened, so preventing unwanted fluid flow (for example, before use of the device, or before the user wishes to process the sample retained on the nib). However, the inventor has determined that the presence of an unopened snap valve can hinder uptake of sample into the nib. The device therefore contains a venting aperture at or adjacent a portion of the body to which the nib is connected or connectable. This allows for capillary flow into the nib when the snap valve is sealed.

In some embodiments, the frangible plug may include a portion of weakness (for example, a thin-walled region) at which the plug will preferentially break when force is exerted. Breakage at this portion will expose a fluid communication channel extending through the remainder of the plug, and hence between the reservoir and the nib. The plug may include a tapered portion, which does not fully restrict the fluid passage, and an interference portion, which does fully restrict the fluid passage but which includes an internal fluid communication channel, with the portion of weakness being located at or near the transition between these two portions. The tapered portion will not include a complete fluid passage therethrough, until snapped.

In some embodiments, the body is formed of a relatively soft plastics material; for example, a polyethylene such as a blow moulded polyethylene. This provides for ease of manufacturing. The frangible plug may be formed of a harder plastics material, for example a hard crystalline polystyrene, which is slightly oversized in comparison to the corresponding portion of the body. This allows a complete interference fit preventing liquid passage. The harder structure of the frangible plug allows it to be cleanly broken or snapped, and in combination with the portion of weakness this directs the snap to the desired location such as at or near the transition between a tapered portion and the interference portion.

The venting aperture may be provided in the body (for example, as a pinhole opening o similar) located towards the end of the nib nearest the reservoir (as opposed to the far end of the nib, which will be that part exposed to sample). Preferably the aperture is at a region of the body which overlaps with the nib, although in some embodiments the aperture may be located above this part of the body (that is, closer to the reservoir than the end of the nib). In some embodiments, the venting aperture may be combined with a pin, a collar, or similar to help to retain the nib in position. In preferred embodiments, the aperture is located between around half and three quarters of the distance along the length of the nib, measured from the end furthest from the reservoir. In some embodiments, the venting aperture includes a groove or other feature along the length of the nib so as to provide airflow between the nib and the body. This may be provided in combination with an aperture provided in the body, or separately.

The reservoir is typically located within the body (for example, as a manually operable bulb). The reservoir may be operable to push fluid towards the nib and/or to withdraw fluid from the nib. Preferably the reservoir is operable by manually squeezing the reservoir, although other options are available - for example, a plunger, a button, a lever, and the like. In some embodiments the reservoir is pressurised to provide fluid to the nib. The reservoir may be removably attached to the nib - this arrangement facilitates provision of a kit form for the device, since the various components may be interchanged or replaced if needed.

The reservoir may contain a liquid reagent formulation for treatment, preferably elution, of the sample obtained on the nib.

The body is preferably in a form suitable for holding in, and manipulation by, the hand. For example, the body may be generally elongate, and preferably has similar dimensions to a writing implement (such as a pen or pencil). In this way, the body and nib combination will feel familiar to a user, and can readily be applied to collection of samples. Whereas the nib is normally expected to be a disposable element, the body may be configured for re-use with new nibs.

The present invention provides a convenient means of obtaining and preparing a sample for analysis, particularly by nucleic acid amplification processes such as PCR, RPA or LAMP, but also potentially by other analytic approaches such as immunodetection. This is achieved, at least in part, by the nib that is used to collect the sample by absorption of liquid or part-liquid, or to wipe a surface containing the biological sample.

In one form, the nib has a chisel- or slanted chisel-shaped working surface to facilitate acquisition of sample at a fine point, at a fine edge, or as a broad stroke. This may be particularly useful in forensics for obtaining samples from various surfaces.

The frangible plug prevents liquid leaving the reservoir until the user is ready to make use of the device. The liquid may be used in various ways; in one embodiment the liquid may be provided to the nib prior to sample collection in order to dampen the nib. This can facilitate sample collection from a dry sample surface (for example, forensic collection from areas which have been touched; or collection from the anterior nares). In other embodiments, the nib can be dry for collection eg of liquid samples such as blood, saliva, urine, etc, and the liquid in the bulb later used for retrieving analyte from the nib. We have also, surprisingly, found that the dry nib is effective in collecting nasal samples (for example, from the anterior nares); meaning that the same format of device can be used for collecting samples from the mouth or the nose.

Note that certain embodiments of the invention need not supply liquid in a squeezable bulb as such. Other formats may be equally suitable for use with the invention; for example, a solid chamber incorporating a plunger, a button, or the like to allow for expulsion of the liquid under pressure. The plunger or button may be indexed to permit more accurate determination of the amount of liquid to be provided.

Suitably, at least one removable cap is provided to shroud the nib before and/or after use. One such cap may be provided over the nib prior to use, and is replaceable after use to protect the sample obtained on the nib.

A cap may be provided with an opening to allow sample-containing droplets to be dispensed under pressure from liquid squeezed from the bulb through the nib.

A reaction chamber - conveniently in the form of the removable cap - may be provided for treatment and/or analysis of the sample obtained on the nib. The device may further comprise a reagent formulation having one or more suitable reagents for such treatment and/or analysis. The reagent formulation is suitably in a freeze-dried or dried-down form to provide room-temperature stability for long-term storage. Such reagents may, for example comprise a protease or detergent, or amplification reagents for the detection of specific nucleic acid targets from the sample (for example, by isothermal nucleic acid amplification, preferably using one of the methods recited in Table 1). In preferred embodiments, the reagent formulation may be provided within the body; for example, located between the bulb and the nib. In such embodiments, in use liquid may be provided to the nib from the bulb and subsequently either withdrawn into the body or flushed through into a separate reaction chamber (eg, the cap) in order to carry sample into the chamber where the reagents may act. The cap may be optically transparent, to permit a user to see the presence of the sample and/or reagents. In some embodiments, the reagents may include means to provide a colour-change reaction under defined conditions (eg, the presence of a particular target in the sample), thereby providing a rapid and easy readout of a sample analysis.

The nib may be provided with a hydrochromic mark to indicate the quantity of aqueous sample acquired. For example, the mark may be provided at a certain point along the length of the nib, so as to indicate when a predetermined amount of aqueous sample has been drawn into the nib. Alternatively, the mark may take the form of a water-sensitive dye throughout the nib which either appears or disappears in proportion to the amount of water present.

Means may be provided to facilitate heating of the nib for drying the sample, for concentrating the sample, or for increasing the volume of sample that can be drawn up. This heating means preferably comprises an element capable of being heated by induction. The nib may include a thermochromic mark to indicate that an appropriate temperature range has been achieved.

The present invention provides a convenient means of obtaining and preparing a sample for analysis (for example, by nucleic acid amplification processes such as PCR or isothermal amplification). This is achieved, in part by the nib that is used to collect the sample by absorption of liquid or part-liquid, or to wipe a surface containing the biological sample. Coupled to this nib through a fluid connection is a squeezable container (or a reservoir which is otherwise operable) that, in one mode of action, provides a liquid to (and through) the nib, releasing analyte (e.g. nucleic acid) for analysis.

As described in more detail below, the nib may comprise an active agent for treatment of the sample obtained on the nib, preferably the nib being functionalised with said active agent. The active agent preferably comprises one or more membrane-disrupting reagents that have the ability to lyse cells (eg bacteria) or viruses, releasing components thereof. For example, the DNA and/or RNA of lysed cells may be collected for further processing or analysis; alternatively, lipids, proteins or peptides may be collected, or subcellular organelles, or cellular fragments including cell membrane or cell wall fragments. Preferably the active agent is non-toxic to humans. One preferred active agent comprises a quaternary ammonium compound (QAC), and a more preferable active agent is cetyl pyridinium chloride, although alternatives (for example, as recited in Table 2, either individually or in combination) may be used. In some embodiments the active agent is in lyophilised or dried-down form, and the reservoir contains aqueous fluid for re-hydrating the active agent. Other uses for the active agent may be to capture certain components that may be inhibitory to the analysis process. One preferred application is for collection of samples for use in lateral flow tests for, for instance, SARS-CoV-2. Typical lateral flow tests for this virus detect the nucleocapsid, rather than the spike protein, so the virus needs to be broken apart to allow for effective testing. In a conventional test this is achieved through agitation or physical disruption of the sample. The present invention instead permits a chemical disruption, which initial data suggests gives an order of magnitude better sensitivity showing improved efficiency at breaking the virus and exposing the nucleocapsid to the antibodies in the lateral flow test device.

In a particularly preferred embodiment, the sample is a liquid biological sample, and the nib contains a dried, non-toxic active agent which lyses bacteria or viruses in the sample. This configuration is particularly suited to obtaining and processing (for example) saliva samples from patients: the saliva is absorbed into the nib via capillary action, and will itself rehydrate the dried active agent allowing lysis to take place and subsequent release of nucleic acids into the nib. The non-toxic nature of the agent permits direct sampling from a patient's mouth, rather than having to use an intermediate collection step.

This preferred embodiment also provides further advantages. The porous structure of the nib will draw a known volume of sample into it, thereby maintaining consistency of sampling across patients; this consistency is enhanced by the use of the dried agent, which reduces dilution of the sample. Furthermore, the use of dried lysis reagents combined with a known sample volume means that the reagents are rehydrated at an optimal concentration for activity while maximising the amount of released material for subsequent analysis. The collected sample can be eluted from the nib for subsequent analysis by washing from the nib using a predetermined volume of reagent contained in the dispenser; again, since the dispenser contains a known volume of reagent, and the nib will absorb a known volume of saliva, consistency of sampling can be maintained and a known dilution of the saliva sample will be obtained. This then has the advantage that accurate quantities of analysis reagents (for example, test chemicals for an isothermal nucleic acid amplification) can be provided with the device, thereby reducing waste as well as ensuring high reliability of analyses.

Further, the amount of dried lysis reagent can be selected so that, after flushing with a known volume from the reservoir, the concentrations of the reagent are low enough so as not to inhibit the subsequent analysis leading to a loss of sensitivity.

Typical agents used for lysis of cells or viruses for release of nucleic acids are toxic, so are not safe to use directly on patients. Such agents will also inhibit nucleic acid amplification or detection, so must be removed from a sample prior to processing. In contrast, the selection of a non-toxic lysis agent permits direct sampling from patients, while also being compatible with direct processing of the sample.

Hence, the invention provides a single device for collection and analysis in which each step of the process of using it is compatible with subsequent steps, as a consequence of the selection of reagents and of the nib and reservoir size.

An optional modification of the device enables concentration of components (for example, nucleic acids) from the sample in order to enhance sensitivity or that enables larger volumes of sample to be processed.

Thus, the invention can provide a single disposable device to obtain a sample, and test for the presence of specific target analyte, such as nucleic acids, in the sample.

In some embodiments of the device, (a) the nib is located in an outlet from the reservoir, from which it projects for sample collection, and for subsequent connection to an inlet of the reaction chamber; or (b) the nib is located in an inlet to the reaction chamber, from which it projects for sample collection, and for subsequent connection to an outlet from the reservoir.

Preferably the nib is removably connectable to the body, and the device is provided with a plurality of replaceable nibs and/or a plurality of bodies and/or a plurality of reaction chambers.

According to another aspect of the present invention there is provided a kit for assembling a sampling device as described here, the kit comprising a selection of nibs and/or bodies, and/or accessory elements such as caps and reagents, from which a sampling device of the present invention can be assembled suited to a particular intended use. This is important where the nature and characteristics of the target analyte are not known, but also where the market for the sampling device is very specific or restricted - e.g. in forensic examination of surfaces.

Although mainly described herein in relation to obtaining and using test samples for analysis of their characteristic nucleic acid content, the present invention can also be applied to immunological (antibody/antigen) testing procedures, or indeed to collection and/or detection of other analytes which may be found in a biological sample where suitable detection reagents exist. For example, lipids, proteins, peptides, subcellular organelles or fragments, cell membrane or cell wall fragments, etc, may all be collected and processed in suitable applications of the invention.

The present invention is able to provide a test sampling device and method for its use, in which the device comprises a porous matrix to absorb test sample in a defined volume, and to expose the sample to an active ingredient that has been pre-functionalised into the nib through a drying process. The sample rehydrates those components as it wicks into the nib. From there, the extracted nucleic acids (or proteins in the case of immunodiagnostics, or lipids, etc) are available immediately, or can dry down during shipping for subsequent elution through buffer exchange by passing buffer back through the nib.

### DRAWINGS AND EXEMPLIFICATION

Fig 1 is a plan view of a device for sample collection and preparation;
Fig 2 shows an exploded plan view of the principal components of the device
Fig 3 shows a plan view of the components of the handle
Fig 4 shows an exploded view of the device of the present invention, including a frangible plug;
Figs 5 and 6 show a detail view of the frangible plug in position within the device; and
Fig 7 shows results from SARS-CoV-2 lateral flow tests carried out with various sample collection techniques.

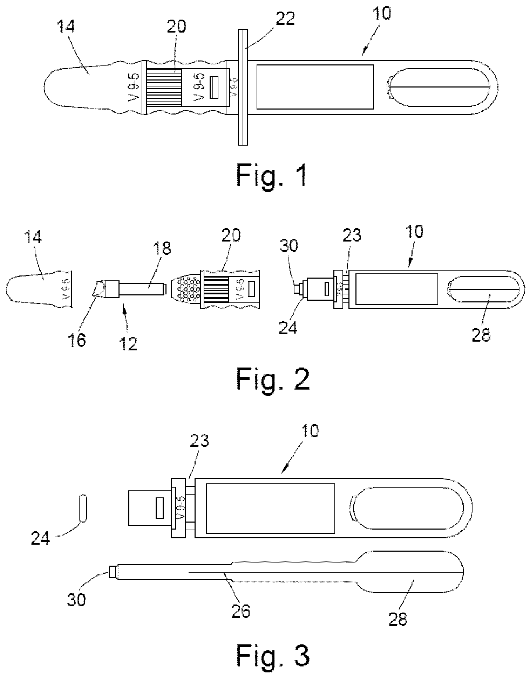

As shown in Figs 1 to 3, an embodiment of a sample collection device (for example, as described in International patent application PCT/EP2021/057315) has the general form and size of a pen, comprising an elongate handle 10 having a sample collection nib 12 at one end covered by a removable protective cap 14.

The nib 12 is carried at the end of a shaft 18, and has a sampling end surface 16 which in this embodiment has a slanting chisel form so as to present a point 17, and an edge 19 flanked by flat surfaces 21.

The shaft 18 is held in a plastics collector component 20 suitably having external mouldings to assist the operator's grip.

The handle 10 contains an internal conduit 26 of flexible material extending lengthwise from an externally exposed squeezable bulb 28 to a stub shaft 30, onto which the collector component 20 is a push fit, clipped in place, and sealed by an O-ring 24 so as to be in fluid connection with the end of nib shaft 18.

The nib 12 is of porous material to facilitate absorption of sample material, and the passage thereto of fluid squeezed from the bulb 28 and/or the passage therefrom of fluid to the bulb 28.

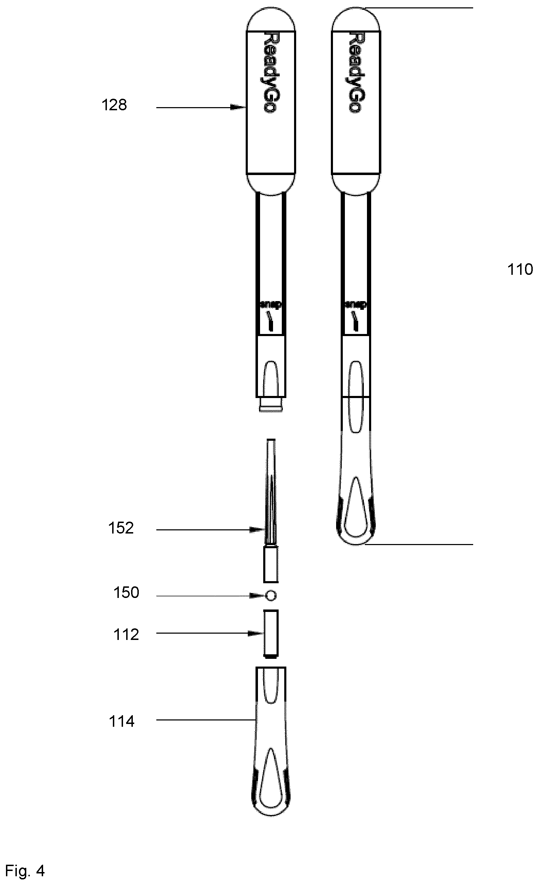

A device according to the present invention is shown in Figure 4. This device also comprises a handle 110 having a nib 112 covered with a cap 114 and a squeezable bulb 128, as described. The device additionally includes a pellet of lyophilised reagent 150 located between the nib 112 and a frangible plug 152.

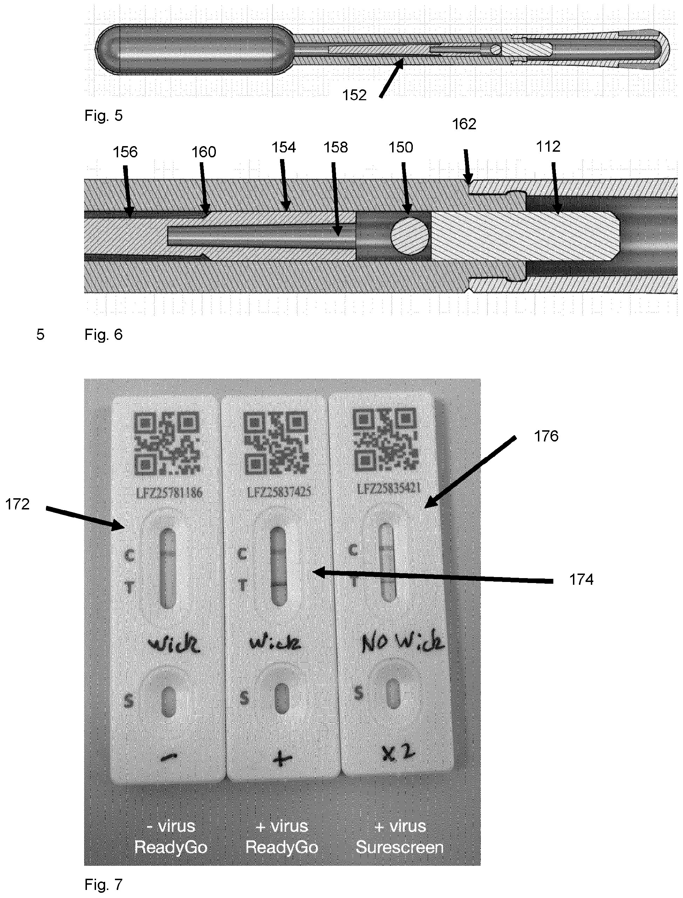

The arrangement of the frangible plug 152 is shown in more detail in Figures 5 and 6. The plug 152 includes a cylindrical portion 154, which sits with an interference fit inside the internal conduit 126, and a tapered portion 156, which allows fluid in the region between the conduit 126 and the outer surface of the tapered portion 156. The cylindrical portion 154 includes a central fluid conduit 158 extending partway along the plug. A region of weakness 160 is formed at the junction between the tapered portion 156 and cylindrical portion 154. A vent aperture 162 is formed in the wall of the device in line with the nib 112, towards the bulb 128 end of the nib.

The handle 110 and bulb 128 are blow moulded from a soft polyethylene, and the frangible plug 152 is a hard crystalline polystyrene which is slightly oversized in comparison to the barrel of the blow moulded part. This allows a complete interference fit preventing liquid passage. The polystyrene's crystalline structure allows it to be cleanly broken or snapped at the region of weakness 160 by a user. When this is done, the central fluid conduit 158 is allowed into fluid communication with the outer surface of the tapered portion 156, thereby permitting fluid flow between the bulb 128 and the nib 112.

The vent aperture 162 is useful to allow sample to be drawn into the nib 112 when the frangible plug is unbroken, as otherwise capillary flow may not be possible given the sealed nature of the unit.

### OPTIONAL AND OPERATIONAL FEATURES

The sampling device of the present invention can be used just to take a sample and hold it for testing later or elsewhere, or it can provide means for performing such testing in the sampling device itself, for example, by LAMP, RPA, or other isothermal amplification methods (see e.g. appended TABLE 1). This would constitute a particularly advantageous use of the device.

### The nib

Suitable nib materials include cotton-based materials, or preferably a plastic based matrix such as PE or PVDF which can be manufactured in a range of densities to absorb and retain biological specimens of different viscosities. The precise detail of the nib construction may vary depending on the specific application for use of the invention, and the skilled person will be able to select appropriate materials and densities. For example, where the sample to be collected is saliva, one such suitable material is a cylindrical PE/PP wick of ~90% porosity.

Marker pen nibs are often made of porous, pressed fibres such as felt or cellulose, or of porous, pressed plastic spheres, to create an open pore structure. The nib in the present invention may suitably be of similar material or structure, and preferably has a defined porosity (void fraction).

The nib can be designed to be task-specific or designed to cope with a range of different tasks. For forensics work it is convenient to have a nib design that allows fine swabbing of a surface for some applications, or a broad surface for sampling a wider area. It may also be convenient to provide a nib which may be used when damp, to collect dry samples for example from surface swabbing or samples from the anterior nares or the epidermis; or to provide a nib which may be used when dry, to collect liquid samples such as blood, urine, saliva, etc.

Felt pen nibs are often designed to allow ink strokes that are either fine or broad from the same single nib. The same design principles are employed here in the nib. The shape presenting the surfaces 16, 17, 19 and 21 is designed especially for collecting sample from a surface, for example in forensics work, where the slanting chisel form of the end surface 16 enables samples to be taken from a surface as a fine point, a fine edge, or as a broad stroke. However, the nib need not have the structural features described and illustrated there. At its simplest it may present merely a blunt or rounded end which is essentially a mere extension of the shaft 18.

The porous form of the nib will provide capillary spaces for absorption of liquid test sample. The porous structure can be designed to draw a known volume of sample fluid, e.g. saliva, into its capillary spaces, thereby regulating the amount of sample taken and making the test more consistent.

The nib may have been treated to form a negatively charged surface and/or treated with an anionic detergent and/or treated with a biocidal agent to provide a means of lysing or removing cellular or viral components of the sample from the target nucleic acid components.

Cellulose fibres have a slight negative (anionic) charge. The hydroxyl groups (-OH) of cellulose can also be partially or fully reacted with various reagents to afford derivatives with useful properties. The nib may thus be provided with a negatively charged surface which binds and retains positively charged ions (such as Fe³⁺) from the test sample, and the bulb used to flow neutral or negatively charged ions and molecules (such as DNA) off the nib and away from cationic components.

Most living cells such as bacteria are surrounded by a fatty lipid bilayer. Lipid composition is not the same across subcellular membranes - mammalian plasma membranes having higher cholesterol and sphingolipid content. Viruses also have envelope lipids which are considered to be the same as the host membranes (phospholipids, sphingolipids, some cholesterol).

There are a number of different chemical groups that destroy the lipid layer of cells and consequently display antibacterial or antiviral properties. If these are used to functionalise the nib of the device, then cells (including bacteria) or viruses that soak into the nib will burst, releasing their DNA and RNA (or other cellular or viral components, such as lipids, proteins, subcellular organelles or fragments, membrane or cell wall fragments, etc).

TABLE 2, appended below, gives examples for functionalising the nib. The mouthwash formulations (shown in the shaded areas) seem particularly useful as they are known to be biosafe and effective, allowing the consumer to place the device into their mouth for self-collection of saliva for testing. For example, cetyl pyridinium chloride (CPC) is the active ingredient of various mouthwashes and has been shown to be a membrane-disrupting agent (Popkin et al, Cetylpyridinium chloride (CPC) exhibits potent, rapid activity against influenza viruses in vitro and in vivo, Pathogens and Immunity, 2017; 2(2):253-69).

Combinations may provide either targeted activity against the lipid bilayer of different organisms so that the same formulation can be used across a range of targets, or it may provide both a targeted preparation with additional antibacterial or antiviral activity to make the sample biosafe after use.

Reagents may be covalently bound to the nib by suitable chemical linkages through functionalisation (e.g. -OH groups), or they may be dried into the nib material and become active on hydration with the sample.

Drying down the formulation onto the nib, minimises dilution of the saliva (or other test sample) and therefore absorption of cleaved material back into the nib matrix. Mouthwash formulations indicated in Table 2 have been shown to act on viruses and bacteria in a matter of seconds. The nib therefore provides the following support functions:
1. A matrix on which to optionally dry down the antiviral and/or antimicrobial formulation, removing the need to dilute the saliva sample with the treatment;
2. Elicit saliva production when inserted into the mouth;
3. Absorb saliva that is elicited;
4. Contain a known volume of saliva.

A saliva sample may be collected and introduced into a tube for subsequent treatment. Or it may be desired to insert the sampler (nib) in the mouth and collect saliva in situ. In that case, the nib may be treated with compounds that have the ability to break open cells (eg bacteria) or viruses, releasing the DNA and/or RNA and/or other cellular or viral components, but are non-toxic and therefore make the product safe to use in that fashion.

### The squeezable bulb

One use of the bulb is as a source of aqueous medium to wash test sample material from the nib and into the cap or other receptacle. It may also have the function of rehydrating dried-down reagent in the cap or in another location within the device.

Suitable liquids that might be held in the bulb include, purified water, milli-q water and buffer (e.g. TRIS-CI / TE buffer).

An initially empty bulb may also be required for use with a sample that has been dried (see below). With the pincer released, the bulb can be used to rehydrate the sample by drawing water or another carrier liquid up through the nib.

It is possible for the device of the present invention to be used without employing the squeezable bulb. For example, in forensics work the users may sometimes want to use their own water, rather than water supplied with the device, in which case the bulb could be supplied empty, or else the pincer left in place, or else the entire handle structure omitted, leaving just the nib and cap assembly for subsequent analysis of the test material captured in the nib. In further embodiments the bulb need not be squeezable, and other means may be provided for pushing fluid from the bulb to and/or from the nib (for example, a plunger, a button, a lever, and so on).

### The cap

As a closed structure, the cap will hermetically seal the nib for safe transport.

Once nucleic acids or other target sample substances are extracted into the nib, they can be deposited into the cap or into test tubes, using liquid supplied from the squeezable bulb. However, variations in cap design, or the provision of one or more additional caps, may provide variable or extended, uses of the device.

For example, one design of cap could have a depositing aperture. Applying liquid under pressure from the squeezable bulb would force liquid through the nib and into the cap. Continuing to apply pressure will force liquid through the aperture in the cap and allow a single drop of extracted material to be deposited into a tube such as a pipettor. Droplet size and size distribution is largely a function of aperture geometry.

In a variant of the device, dried reagent may be provided within the body, for example, in a chamber between the bulb and the nib. Fluid from the bulb can be used to rehydrate the reagent in the chamber, and either push the rehydrated reagent through the nib into the cap, or enter the nib and be drawn back into the chamber carrying the sample, where the reaction can take place.

### Sample drying, and optionally heating

The nib carrying the biological sample will dry naturally over time, determined by the external environment, temperature and humidity. This can be a convenient process if the sample is first collected and sent elsewhere for processing at some point in the future (e.g. next working day). However, natural drying times may be compromised by environmental factors (e.g. low temperature or high humidity), and there may be certain applications where it is preferable for drying to be done more rapidly to facilitate point of care (POC), point of need (PON) or on-the-spot testing.

Drying can be assisted by applying heat to the device, for example by placing the device into a hot space such as a drying oven or hot block. Drying ovens are often not convenient to use, however, because they represent a large dedicated piece of equipment which is not generally transportable outside of a laboratory. So heat may be supplied using a heating element associated with the structure of the device. For example, a collar around the shaft of the nib can be heated, preferably by a noncontact induction field. The induction heater could be supplied as a separate small device which would be portable and convenient for limited resource settings such as outside of the laboratory or within a small laboratory environment.

The heating element may extend along the length of the shaft, or it may be positioned towards the end of the shaft distal from the nib as shown in the drawings. Any suitable induction material may be used, and may be a non-metal, such as carbon fibre.

Heating can also be used to thermally lyse the biological material in the sample contained in the nib. This may be of importance to release nucleic acids so that they are accessible to molecular amplification, and to render the sample in the device biologically safe.

Reagents may be developed by heating in a controlled manner according to the type of test being performed.

### Sample concentration

Apart from heating the nib to dry the sample, heat may be used to concentrate the sample, or to increase the volume of sample that can be drawn up using evaporation. Liquid in the nib is drawn up to the heat front via evaporation of the liquid phase at the end. This front will therefore create a continuous capillary draw of sample from the wetter nib. If the non-heated nib sits in a volume of liquid, for example in a reservoir, then as liquid is evaporated at the heated end, all the liquid will finally be drawn into the nib. In this way liquid in the nib will become concentrated until the supply of liquid sample is exhausted. This approach could also be used to draw a substantial sample of saliva by placing the nib under the tongue and applying the induction heating. Evaporated water is lost to the local environment as vapour (steam). Because volumes are small (<200µL) and evaporated over a few minutes there is minimal condensate.

Heating can therefore be used not only to speed the drying process, but also to allow more sample to flow into the nib, thereby concentrating the analyte from a quantity of liquid larger than the volume within the nib itself.

### Sample release

The nib may need to be dried before releasing the extracted materials (see above).

Nucleic acids are negatively charged molecules. If the nib is also negatively charged then nucleic acids are repelled by the same charge and easily released into a mobile phase as it is forced through the nib.

Depending on the surface chemistry of the nib, the nib may also be positively charged in order to bind nucleic acids. This charge may be altered (e.g. by buffer exchange) to facilitate release of bound nucleic acids in the mobile phase. Often a change in pH will release certain charged molecules.

Other cellular or viral components may of course be collected; suitable chemistries and methods are known for permitting sample retention and subsequent release depending on the nature of the component and the nib.

### Feedback on use

The nib can be treated to provide feedback information on use. For example, indicator dyes can be employed on the nib to show when and/or how the nib has been used to obtain a liquid sample.

Thermochromic inks change colour when they have been exposed to certain temperatures. Hydrochromic inks change colour when wet. It may be desirable to use these in certain scenarios to indicate when the device has been used, and if the device has been used correctly. For example, these can be applied to the nib to provide a visual indication that the nib has been exposed to enough liquid, or if the nib has been heated up to for example >90°C.

Thus, by treating a distal part of the nib with a hydrochromic ink, colour changes will occur once the nib has received enough liquid to fill the nib to that marker position. This can be useful to give a positive indication that the device has been used, and that enough material has been collected.

The porous structure of the nib can be designed to draw a known volume of sample fluid, e.g. saliva, into its capillary spaces, thereby regulating the amount of sample taken and making the test more consistent.

Surface treatment chemistries can add lysing capabilities to the nib. Some biocidal chemistries by virtue of their mode of action also provide lysing capabilities. Other chemistries offer a means of capturing certain chemical groups which can be important for samples containing high concentrations of inhibitory components such as urine.

### Lysing chemistry

International patent applications WO00/62023 and WO02/16383 to Whatman, Inc, describe methods for lysing cells and isolating nucleic acids using coated filter medium (FTA-treated nitrocellulose), which is coated with an anionic detergent, for example SDS. The coating lyses the cells, and the FTA-treated filter adsorbs nucleic acids. The coating is not covalently bound to the filter and may be removed by washing. Nucleic acids can be eluted from the filter for subsequent processing. These materials may be useful in the practice of the present invention.

### Biocidal agents

European Patent application EP2855677A1 describes a paper-based system that works at room temperature, is capable of rupturing bacterial, fungal and viral cells, and releases nucleic acids into solution for direct-to-PCR analysis by functionalising the surface with a biocidal agent. The biocidal agent preferably comprises multiple functional groups. The functional groups preferably include a binding component which is involved in binding the agent to the substrate; a hydrophobic component; and a charged component. The hydrophobic component can interact with and penetrate the cell wall or cell membrane. These materials may be useful in the practice of the present invention.

In preferred embodiments, the hydrophobic component may be an alkyl chain, for example C5-C30 alkyl, preferably C10-C20 alkyl. As the alkyl chain penetrates the delicate cell wall, the wall is weakened and punctured. The charged component is preferably positively charged, and can attract a charged cell wall, and can disrupt ion flow and homeostasis on contacting a cell membrane, thereby helping to disrupt the cell and release the nucleic acids. The charged component is preferably a quaternary ammonium group. The binding component may comprise a hydroxyl group.

In preferred embodiments, the functional groups are preferably an alkyl chain (the hydrophobic component), a silyl group (the binding component), and an ammonium chloride group (the charged component). Preferred biocidal agents include silylated quaternary ammonium compounds (SiQACs); in particular 3-(trimethoxysilyl) propyldimethyloctadecyl ammonium chloride (3-TPAC). Other biocidal agents include benzyl ammonium chlorides. The lethal mode of action of SiQACs is generally accepted to proceed by adsorption of the positively charged molecule onto the negatively charged cell surface, disruption of the cell membrane by a lipophilic chain on the SiQAC molecule, and diffusion through the membrane leading to cell lysis.

The skilled person will be aware of other suitable biocidal agents that may be used. The selection of a particular agent will be guided by the presence of the preferred functional groups described above, and the nature of the intended biological sample - for example, where the sample to be processed is a mammalian cellular sample, then there is no cell wall to penetrate, and other functional groups may be appropriate.

For a review of other compositions which may be used, see "Antimicrobial Polymers in Solution and on Surfaces" (Siedenbiedel and Tiller (2012) Polymers, (4) 46-71). Specific examples of biocidal agents which may be used in the present invention include:
(i) Telechelic poly-(2-alkyl-1, 3-oxazolines).
(ii) Cellulose based fibres with an antimicrobial DDA group grafted via PEtOx, which kills approaching microbial cells on contact (Bieser et al., Contact-Active Antimicrobial and Potentially Self-Polishing Coatings Based on Cellulose, 2011, Macromol. Biosci., 11, 111-121).
(iii) Saponins (steroid or triterpenoid glycosides), common in a large number of plants, and have long been known to have a lytic action on erythrocyte membrane, many saponins being known to be antimicrobial (Francis et al., British Journal of Nutrition. 2002, (88) 587-605). Extensive research has been carried out into the membrane-permeabilising properties of saponins. These structurally diverse compounds have also been observed to kill protozoans and to act as anti-fungal and antiviral agents. Isolated cell membranes from human erythrocytes when treated with saponin developed pores of 40-50Å diameter, as against the 80Å pores produced in artificial membranes (Seeman et al. Structure of membrane holes in osmotic and saponin hemolysis, 1973, Journal of Cell Biology (56), 519-527).

### Binding components

Chitosan-modified Fusion 5 filter paper (unmodified ones purchased from GE Healthcare) have been successfully developed for DNA extraction and concentration (Francis et al., British Journal of Nutrition, 2002, (88) 587-605). The modified filter paper employs two separate mechanisms: the physical entanglement of long-chain DNA molecules with the fibre matrix of the filter paper, and the electrostatic adsorption of DNA to the chitosan-modified filter fibres. This enables a bonding and capture of DNA to the fibres and subsequent washing of inhibitors prior to PCR.

### USE CASE SCENARIOS

### 1. Sample collection

The sample collector can be used on its own as part of a large scale or centralised testing process. For example, the 2020 SARS-CoV-2 pandemic has seen various mass testing strategies implemented worldwide. These tests have generally been done within a centralised laboratory network, processing hundreds of thousands of samples each day.

Using the sample collector with a nib that has been functionalised with compounds known to break open the lipid capsid of the virus, it is possible to collect samples at home for subsequent posting back through the post. This reduces the need to take nasopharyngeal samples which requires a skilled person to take effectively. The sample collector may be provided with a gasket part that interfaces with the plastic pipette tips of automated liquid handling units in robotic sample processing laboratories. In some embodiments, the cap may be designed to receive a collected sample which is subsequently eluted from the nib using liquid from the bulb; the cap can then be removed, sealed, and sent for further processing. Alternatively, the nib itself may be removed and sent for further processing without elution.

The additional benefit is that virus is broken up and the RNA released into the tip as the sample is handled and transported back to the test facility. Elution of pre-lysed material back at the lab allows a simpler test of the sample without the need for extraction in the lab.

This same approach may be used for other targets which require more centralised testing.

The nib may be of different materials which offer different characteristics according to task. There are two main plastics that are especially useful, polyvinylidene fluoride (PVDF) and polyethylene (PE).

PVDF is a highly non-reactive thermoplastic fluoropolymer produced by the polymerization of vinylidene difluoride. PVDF is a speciality plastic used in applications requiring the highest purity, as well as resistance to solvents, acids and hydrocarbons. It is normally manufactured into nibs for pens which are made using the sintering process. They generally form nibs that have good flow characteristics and can be of differing 'hardness'. This is especially useful if the nib is required to be used in a physical process such as wiping a surface or crushing a sample in order to release an exudate to be then drawn into the nib and tested.

PE is a lightweight, durable thermoplastic with variable crystalline structure. It is one of the most widely produced plastics in the world (tens of millions of tons are produced worldwide each year). PE is used in applications such as films, tubes, plastic parts, laminates, etc. PE has been used to create materials that have an open structure that is particularly useful in collection of saliva. The manufacture can be controlled to provide materials within different retention and flow rates.

### 2. Sample collection and dispense

This combination allows sample collection and elution of extracted material into another test such as a molecular diagnostic or an immunodiagnostic. A known volume is drawn into the nib material where the specific functionalisation acts on microorganisms contained within the sample matrix. The extracted material is washed off the tip using the reagent contained in the dispenser/bulb element of the device. Pushing the rehydration buffer from the dispenser through the nib washes the saliva into the test cap. Control by volume creates a known dilution of the saliva since the nib absorbs a defined volume (e.g. 25µL) and the dispenser/bulb has a known volume (e.g. 200µL).

### 3. Sample collection, dispense and test

This combination allows both professional point-of-care, and consumer over-the-counter testing. Collection of saliva by sucking on the end of the nib generates saliva which is collected into the nib. After 5 minutes sufficient saliva is collected, and the targeted bacteria are broken down by the antimicrobials functionalised into the nib matrix. This releases the nucleic acids into the aqueous phase. Pushing the rehydration buffer from the dispenser/bulb through the nib washes the saliva into the test cap. Control by volume creates a known dilution of the saliva since the nib absorbs a defined volume (e.g. 25µL) and the dispenser/bulb has a known volume (e.g. 200µL). Test chemicals, preferably for an isothermal amplification such as recombinase polymerase amplification, are rehydrated and over a period of time will develop a colour change if the target DNA is present in the nib. This may be of a simple colour change (e.g. yellow to red) using a pH indicator or may be development of a fluorescent colour (e.g. blue to green).

### SOME SPECIFIC APPLICATIONS

The applications of the present invention are broad since the device provides all of the steps required from sampling, sample processing, elution and testing. Examples of specific applications are given below;

Periodontal disease, or periodontitis, is caused by a dysbiosis within the dental plaque microbial community causing the disruption of tissue homeostasis (Hajishengallis et al, Beyond the red complex and into more complexity: the polymicrobial synergy and dysbiosis (PSD) model of periodontal disease etiology, Mol Oral Microbiol. 2012; 27:409-419). It affects 10-15% of adults and is the most common cause of tooth loss worldwide. A test that could be bought OTC would allow people to better track the abundance of bacteria causing periodontal disease at home.

Providing a system capable of reporting a positive if any of the main causes of periodontal disease is present in the sample would be of particular use as an OTC device - for example, the following six species may be considered indicative of periodontal disease:
*Porphyromonas gingivalis,* ATCC33277
*Actinobacillus actinomycetemcomitans,* ATCC29523
*Fusobacterium nucleatum,* No. 2
*Tannerella forsythensis,* ATCC43937
*Prevotella intermedia,* ATCC25611
*Streptococcus anginosus,* ATCC33397

*Porphyromonas gingivalis* and *Actinobacillus actinomycetemcomitans* have been shown to be key bacteria responsible causing a chronic inflammatory disease of the periodontium, the tissues that surround and support the teeth. Both species were found in 33% and 44%, respectively, of subjects with moderate periodontitis and in 60% and 40%, respectively, of the subjects with advanced periodontitis. (Troil-Lindén et al, (1995). Salivary Levels of Suspected Periodontal Pathogens in Relation to Periodontal Status and Treatment. Journal of Dental Research).

Some cancers: Around 1 in 4 mouth cancers and 1 in 3 throat cancers are HPV-related, but in younger patients most throat cancers are now HPV-related. A study carried out in 2009-10 concluded that 1 in 10 American men and less than 4 in 100 American women had HPV infection in the mouth. Another study published in 2017 found that in America, 6 in 100 men and 1 in 100 women carried potentially cancer-causing types of HPV in their mouth.

There is solid evidence to support the use of saliva as a subject for diagnostic testing for the detection of HPV DNA in oropharyngeal cancer (OPC) patients. (Rosenthal et al. Detection of HPV related oropharyngeal cancer in oral rinse specimens, Oncotarget 2017;8:109393-109401 and Chai et al, A pilot study to compare the detection of HPV-16 biomarkers in salivary oral rinses with tumour p16(INK4a) expression in head and neck squamous cell carcinoma patients. BMC Cancer. 2016;16:178).

Furthermore, the detection of HPV DNA in saliva collected by different methods (drool or oral rinse) yields comparable results and shows good sensitivity for the detection of HPV, again supporting the feasibility of using saliva as a diagnostic medium for OPC. (Tang et al, High-risk human papillomavirus detection in oropharyngeal cancers: Comparison of saliva sampling methods, Head Neck. 2018)

Providing a system capable of reporting a positive if any of the main cancer-causing genotypes of HPV were present in a saliva sample would be of particular use as an OTC device.

White spot disease in penaeid shrimp is caused by the white spot syndrome virus (WSSV). It is the most economically important disease of farmed warm-water shrimp, causing extensive economic losses estimated from $8 to $15 billion since its emergence in the 1990s. Early diagnosis of disease is critical in the management of outbreaks and to avoid crop losses.

Providing a system capable of first crushing a portion of shrimp to release the haem containing WSSV, then absorbing the haem into the nib for nucleic acid extraction, elution and test would be of particular use as a device for agricultural diagnostics.

### EXAMPLE - LATERAL FLOW TESTING

A device as described was used to collect surrogate sample for use in a test lateral flow assay for SARS-CoV-2. In this example, the nib was functionalised with citral, in order to release nucleocapsid from any viral particles, and the reservoir was filled with PBS buffer containing BSA, and tween.

The surrogate sample was SARS-CoV-2 spiked into pig mucus, which is a recognised surrogate for testing of lateral flow devices. A comparison was made between using positive and negative samples collected with the device as described herein, and a positive sample collected with a conventional lateral flow test device kit (from Surescreen Ltd).

After sample collection and processing, the sample was then added to lateral flow tests. Results are shown in Figure 7 - from left to right, test kit 172 was a negative sample collected with the device described herein; test kit 174 was a positive sample collected with the device described herein; and test kit 176 a positive sample collected with the conventional lateral flow test device kit. Further, test kit 176 was tested with twice the amount of sample obtained from processing. It can be seen that test kit 174 provides a strong signal after collection and processing using the chemically functionalised nib (which releases nucleocapsid through chemical means), comparable with the signal from the conventional kit which is released through physical means.

This demonstrates that the device as described herein is effective in releasing biological material without physical disruption in a form suitable for downstream testing and analysis.

**Table 1: Summary of isothermal amplification methods**

| | |
|---|---|
| NASBA | *Nucleic acid sequence-based amplification* is a method used to amplify RNA |
| LAMP | *Loop-mediated isothermal amplification* is a single tube technique for the amplification of DNA. It uses 4-6 primers, which form loop structures to facilitate subsequent rounds of amplification |
| HAD | *Helicase-dependent amplification* uses the double stranded DNA unwinding activity of a helicase to separate strands for *in vitro* DNA amplification at constant temperature |
| RCA | *Rolling circle amplification* starts from a circular DNA template and a short DNA or RNA primer to form a long single stranded molecule |
| MDA | *Multiple displacement amplification* is a technique that initiates when multiple random primers anneal to the DNA template and the polymerase amplifies DNA at constant temperature |
| WGA | When MDA is used to amplify DNA from a whole genome of a cell it is called *whole genome amplification.* (Other methods of WGA include MALBAC, LIANTI, DOP-PCR) |
| RPA | *Recombinase polymerase amplification* is a low temperature DNA and RNA amplification technique. |

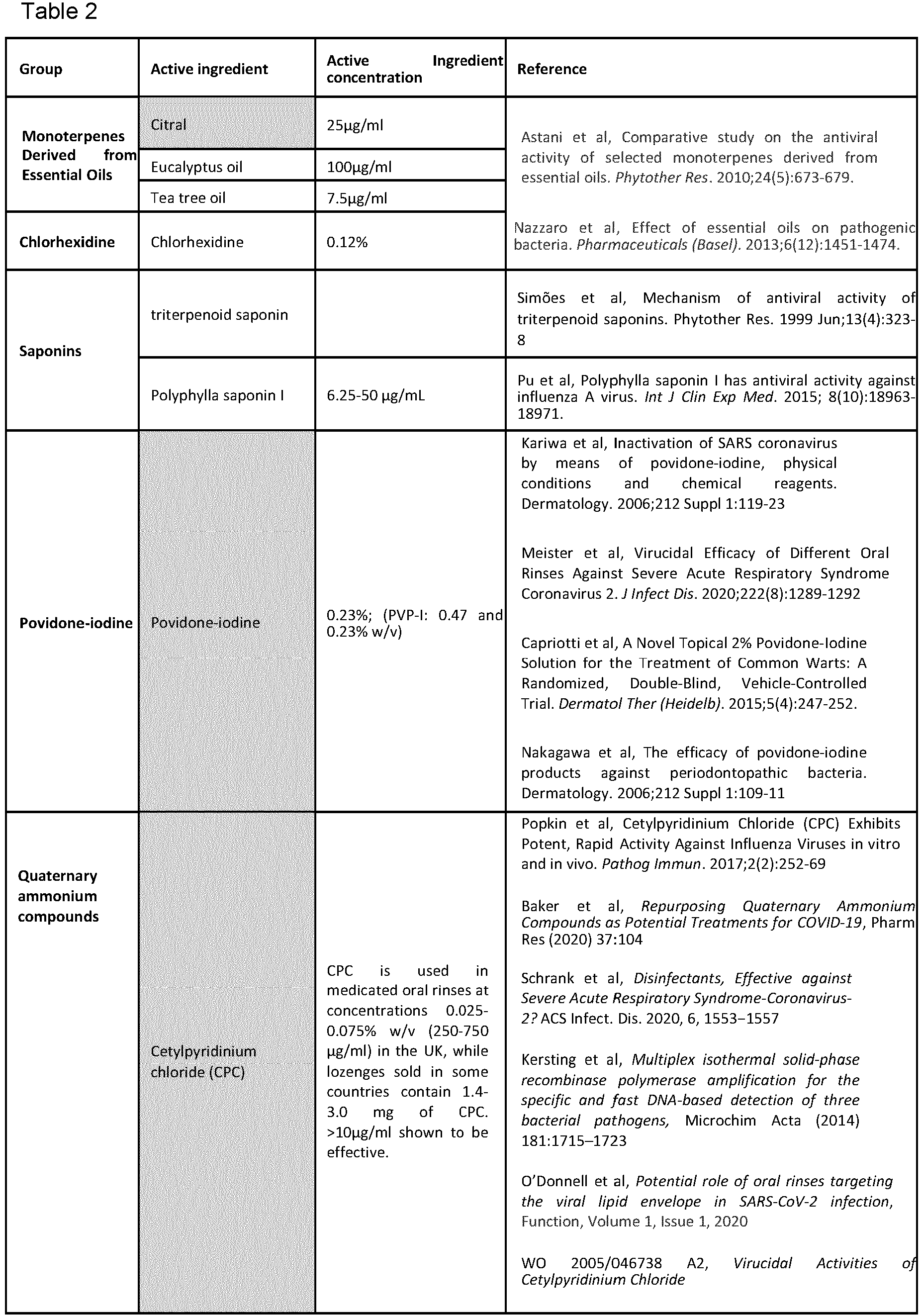

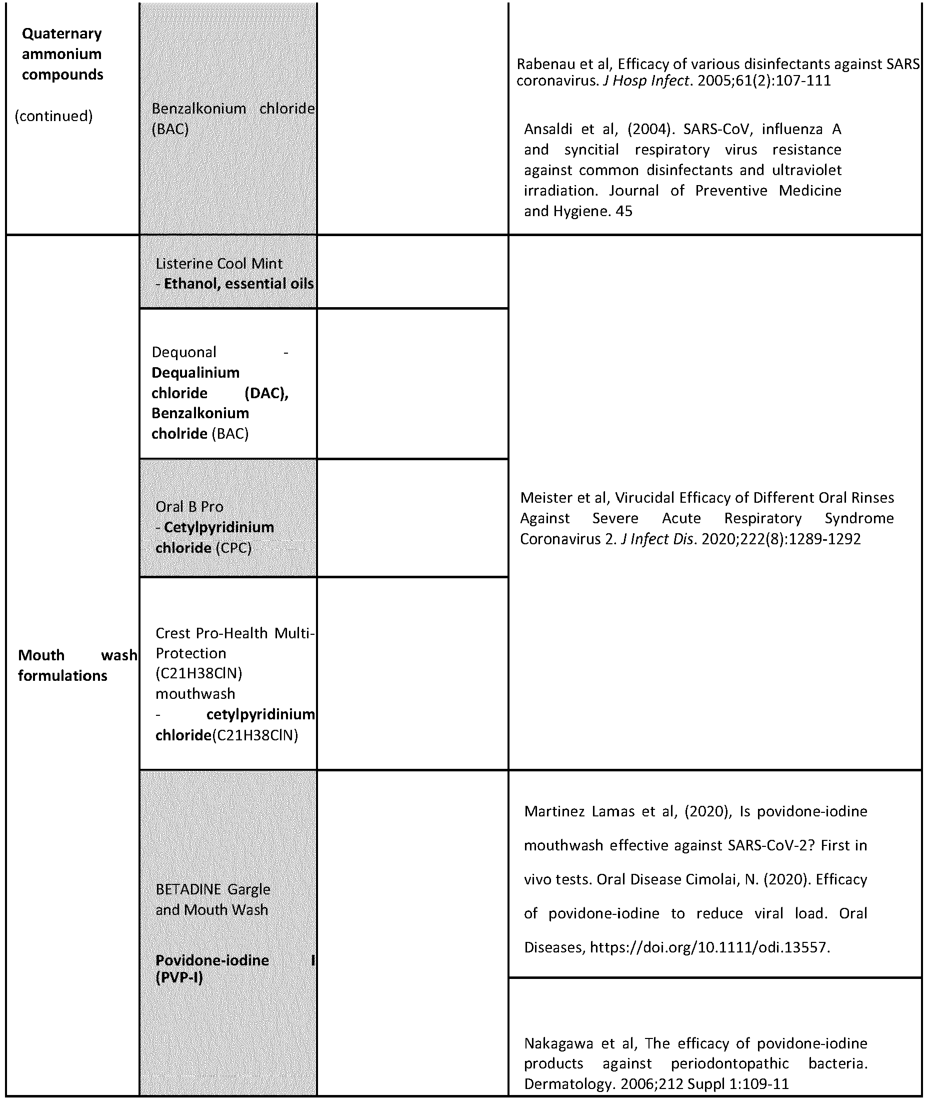

## Claims

1. A device for obtaining biological samples for analysis, comprising:
(a) a nib (112) having a working surface exposed or exposable for acquiring a biological sample, and having also a porous structure suitable for the absorption of biological sample matter thus acquired;
(b) a body (110) having a form suitable for holding in, and manipulation by, the hand, and wherein the nib (112) is connected or connectable to the body (110); and
(c) a reservoir (128) located within the body (110) and adapted for fluid communication with the nib (112) to provide the passage of fluid through the nib (112); wherein the device further comprises:
(d) a plug (152) located within a fluid passage between the reservoir (128) and the nib (112) to prevent passage of fluid between the reservoir and the nib, said plug (152) being frangible so as to open a fluid passage and thereby permit passage of fluid between the reservoir (128) and the nib (152); and **characterised in that** the device further comprises a venting aperture (162) located at a region of the body which overlaps the nib when the nib is connected to the body, or above this region, the aperture allowing for capillary flow into the nib when the fluid passage is sealed by the plug (152).

2. A device according to claim 1 wherein the frangible plug (152) includes a portion of weakness (160) at which the plug will preferentially break when force is exerted, to expose a fluid communication channel extending through the remainder of the plug, and hence between the reservoir and the nib; and/or wherein the frangible plug (152) comprises a tapered portion (156), which does not fully restrict the fluid passage, and an interference portion (154), which does fully restrict the fluid passage.

3. A device according to any preceding claim wherein the body (110) is formed of a softer plastics material; and the frangible plug (152) is formed of a harder plastics material

4. A device according to any preceding claim wherein the venting aperture (162) is combined with a pin, a collar, or similar to retain the nib (112) in position in the device; and/or wherein the venting aperture (162) comprises a groove provided along the length of the nib (112) so as to provide airflow between the nib and the body; and/or wherein the venting aperture (162) comprises an opening formed in the body.

5. A device according to any preceding claim wherein the reservoir (128) is operable to push fluid towards the nib (112) and/or to withdraw fluid from the nib; preferably wherein the reservoir (128) is operable by manually squeezing the reservoir.

6. A device according to any one of the preceding claims, wherein the nib (112) is associated with a hydrochromic mark to indicate the quantity of aqueous material acquired.

7. A device according to any one of the preceding claims, wherein the reservoir (128) contains a liquid reagent formulation for treatment, preferably elution, of the sample obtained on the nib (112).

8. A device according to any one of the preceding claims, wherein the nib (112) comprises an active agent for treatment of the sample obtained on the nib, and preferably the nib is functionalised with said active agent, preferably wherein the active agent comprises one or more membrane-disrupting reagents that have the ability to lyse bacteria or viruses.

9. A device according to any one of the preceding claims, further comprising a reaction chamber (114) to receive fluid supplied from the reservoir (128) and discharged from the nib (112) after contact with the sample, preferably wherein the reaction chamber (114) can be removably located so as to cover the nib (112).

10. A device according to any preceding claim, comprising a reagent formulation (150) for treatment of the sample, preferably wherein the reagent formulation (150) is in a lyophilised or dried-down form.

11. A device according to claim 10, wherein the reagent formulation (150) comprises one or more reagents for the detection of specific nucleic acid targets in the sample, preferably wherein said reagent formulation (150) comprises reagents for isothermal amplification of nucleic acid, and more preferably wherein the isothermal amplification is recombinase polymerase amplification.

12. A device according to any of claims 10 or 11 wherein the reagent formulation (150) provides a visual signal in the event of detection of said specific nucleic acid targets.

13. A device according to claim 10 wherein the reagent formulation (150) comprises one or more reagents for the detection of specific proteins, peptides, and/or lipids in the sample, preferably wherein the reagent formulation (150) comprises one or more reagents for immunoassay-based detection of a target in the sample.

14. A device according to any preceding claim, provided in kit form.

15. Use of a device according to any preceding claim in collection and preparation of a viral sample for use in a lateral flow assay, preferably wherein the viral sample is a SARS-CoV-2 sample.

## Patentansprüche

1. Vorrichtung zum Gewinnen biologischer Proben zur Analyse, Folgendes umfassend:
(a) eine Spitze (112) mit einer Arbeitsfläche die zum Erlangen einer biologischen Probe freiliegt oder freilegbar ist, und außerdem mit einer porösen Struktur, die zur Absorption von so erlangtem biologischem Probenmaterial geeignet ist;
(b) einen Hauptteil (110) mit einer Form, die zum Halten in der und zum Handhaben durch die Hand geeignet ist, und wobei die Spitze (112) mit dem Hauptteil (110) verbunden oder verbindbar ist; und
(c) einen Behälter (128), das sich in dem Hauptteil (110) befindet und zur Fluidverbindung mit der Spitze (112) eingerichtet ist, um den Durchlass von Fluid durch die Spitze (112) bereitzustellen; wobei die Vorrichtung ferner Folgendes umfasst:
(d) einen Pfropf (152), der sich in einem Fluiddurchlass zwischen dem Behälter (128) und der Spitze (112) befindet, um das Hindurchlassen von Fluid zwischen dem Behälter und der Spitze zu verhindern, wobei der Pfropf (152) zerbrechlich ist, so dass ein Fluiddurchlass geöffnet und dadurch das Hindurchlassen von Fluid zwischen dem Behälter (128) und der Spitze (152) ermöglicht wird, und **dadurch gekennzeichnet, dass**
die Vorrichtung ferner eine Lüftungsöffnung (162) umfasst, die sich an einem Bereich des Hauptteils, der die Spitze überlagert, wenn die Spitze mit dem Hauptteil verbunden ist, oder über diesem Bereich befindet, wobei die Öffnung einen Kapillarfluss in die Spitze gestattet, wenn der Fluiddurchlass durch den Pfropf (152) verschlossen ist.

2. Vorrichtung nach Anspruch 1, wobei der zerbrechliche Pfropf (152) eine Schwachstelle (160) einschließt, an der der Pfropf beim Ausüben von Kraft vorzugsweise brechen wird, um einen Fluidverbindungskanal freizugeben, der sich durch den Rest des Pfropfs und somit zwischen dem Behälter und der Spitze erstreckt; und/oder wobei der zerbrechliche Pfropf (152) einen abgeschrägten Abschnitt (156) umfasst, der den Fluiddurchlass nicht vollständig einschränkt, und einen Eingriffsabschnitt (154), der den Fluiddurchlass durchaus vollständig einschränkt.

3. Vorrichtung nach einem der vorhergehenden Ansprüche, wobei der Hauptteil (110) aus einem weicheren Kunststoff gebildet ist und der zerbrechliche Pfropf (152) aus einem härteren Kunststoff gebildet ist.

4. Vorrichtung nach einem der vorhergehenden Ansprüche, wobei die Lüftungsöffnung (162) mit einem Stift, einer Manschette oder ähnlichem kombiniert ist, um die Spitze (112) in der Vorrichtung in Position zu halten; und/oder wobei die Lüftungsöffnung (162) eine Rille umfasst, die über die Länge der Spitze (112) bereitgestellt ist, so dass ein Luftstrom zwischen der Spitze und dem Hauptteil bereitgestellt ist; und/oder wobei die Lüftungsöffnung (162) eine Öffnung umfasst, die in dem Hauptteil gebildet ist.

5. Vorrichtung nach einem der vorhergehenden Ansprüche, wobei der Behälter (128) betätigbar ist, um Fluid hin zu der Spitze (112) zu drücken und/oder Fluid aus der Spitze zu ziehen; wobei der Behälter (128) vorzugsweise durch manuelles Zusammendrücken des Behälters betätigbar ist.

6. Vorrichtung nach einem der vorhergehenden Ansprüche, wobei der Spitze (112) eine hydrochrome Markierung zugeordnet ist, um die Menge erforderlichen wässrigen Materials anzugeben.

7. Vorrichtung nach einem der vorhergehenden Ansprüche, wobei der Behälter (128) eine flüssige Reagensformulierung zum Behandeln, vorzugsweise Eluieren, der an der Spitze (112) gewonnenen Probe enthält.

8. Vorrichtung nach einem der vorhergehenden Ansprüche, wobei die Spitze (112) einen Wirkstoff zum Behandeln der an der Spitze gewonnenen Probe umfasst und die Spitze vorzugsweise mit dem Wirkstoff funktionalisiert ist, wobei der Wirkstoff vorzugsweise ein oder mehrere membranbrechende Reagenzien umfasst, welche die Fähigkeit besitzen, Bakterien oder Viren zu lysieren.

9. Vorrichtung nach einem der vorhergehenden Ansprüche, ferner eine Reaktionskammer (114) zur Aufnahme von Fluid umfassend, das von dem Behälter (128) zugeführt und nach Kontakt mit der Probe aus der Spitze (112) abgelassen wird, wobei die Reaktionskammer (114) vorzugsweise abnehmbar angeordnet sein kann, so dass die Spitze (112) bedeckt ist.

10. Vorrichtung nach einem der vorhergehenden Ansprüche, eine Reagensformulierung (150) zur Behandlung der Probe umfassend, wobei die Reagensformulierung (150) vorzugsweise in lyophilisierter oder getrockneter Form vorliegt.

11. Vorrichtung nach Anspruch 10, wobei die Reagensformulierung (150) ein oder mehrere Reagenzien zum Nachweisen spezifischer Nukleinsäureziele in der Probe umfasst, wobei die Reagensformulierung (150) vorzugsweise Reagenzien zur isothermen Amplifikation der Nukleinsäure umfasst und wobei die isotherme Verstärkung bevorzugter eine Rekombinase-Polymerase-Amplifikation ist.

12. Vorrichtung nach einem der Ansprüche 10 oder 11, wobei die Reagensformulierung (150) im Falle des Nachweises der spezifischen Nukleinsäureziele ein visuelles Signal bereitstellt.

13. Vorrichtung nach Anspruch 10, wobei die Reagensformulierung (150) ein oder mehrere Reagenzien zum Nachweisen spezieller Proteine, Peptide und/oder Lipide in der Probe umfasst, wobei die Reagensformulierung (150) vorzugsweise ein oder mehrere Reagenzien zum immun-assay-basierten Nachweis eines Ziels in der Probe umfasst.

14. Vorrichtung nach einem der vorhergehenden Ansprüche, bereitgestellt in Form eines Kits.

15. Verwendung einer Vorrichtung nach einem der vorhergehenden Ansprüche beim Sammeln und Präparieren einer viralen Probe zur Verwendung in einem Lateral-Flow-Assay, wobei die virale Probe vorzugsweise eine SARS-CoV-2-Probe ist.

## Revendications

1. Dispositif pour obtenir des échantillons biologiques à des fins d'analyse, comprenant :
(a) une pointe (112) ayant une surface de travail exposée ou pouvant être exposée pour prélever un échantillon biologique, et ayant également une structure poreuse appropriée pour l'absorption de la matière de l'échantillon biologique ainsi prélevé ;
(b) un corps (110) ayant une forme appropriée pour la tenue et la manipulation manuelle, et dans lequel la pointe (112) est connectée ou peut être connectée au corps (110) ; et
(c) un réservoir (128) situé à l'intérieur du corps (110) et adapté à la communication fluidique avec la pointe (112) pour assurer le passage de fluide à travers la pointe (112) ; dans lequel le dispositif comprend en outre :
(d) un bouchon (152) situé à l'intérieur d'un passage de fluide entre le réservoir (128) et la pointe (112) pour empêcher le passage de fluide entre le réservoir et la pointe, ledit bouchon (152) étant destiné à la rupture de manière à ouvrir un passage de fluide et à permettre ainsi le passage de fluide entre le réservoir (128) et la pointe (152) ; et **caractérisé en ce que**
le dispositif comprend en outre une ouverture d'aération (162) située dans une région du corps qui chevauche la pointe lorsque la pointe est connectée au corps, ou au-dessus de cette région, l'ouverture permettant l'écoulement capillaire dans la pointe lorsque le passage de fluide est scellé par le bouchon (152).

2. Dispositif selon la revendication 1, dans lequel le bouchon destiné à la rupture (152) inclut une partie de faiblesse (160) au niveau de laquelle le bouchon se rompt de préférence lorsqu'une force est exercée, pour exposer un conduit de communication fluidique s'étendant à travers le reste du bouchon, et donc entre le réservoir et la pointe ; et/ou dans lequel le bouchon destiné à la rupture (152) comprend une partie conique (156), qui ne restreint pas intégralement le passage de fluide, et une partie d'interférence (154), qui restreint intégralement le passage de fluide.

3. Dispositif selon l'une quelconque des revendications précédentes, dans lequel le corps (110) est formé en une matière plastique plus molle et le bouchon destiné à la rupture (152) est formé en une matière plastique plus dure.

4. Dispositif selon l'une quelconque des revendications précédentes dans lequel l'ouverture d'aération (162) est combinée à une goupille, un collier ou semblable pour maintenir la pointe (112) en position dans le dispositif ; et/ou dans lequel l'ouverture d'aération (162) comprend une rainure prévue sur la longueur de la pointe (112) de manière à assurer un flux d'air entre la pointe et le corps ; et/ou dans lequel l'ouverture d'aération (162) comprend un orifice formé dans le corps.

5. Dispositif selon l'une quelconque des revendications précédentes, dans lequel le réservoir (128) peut s'utiliser pour pousser du fluide vers la pointe (112) et/ou pour retirer du fluide de la pointe ; de préférence, dans lequel le réservoir (128) peut s'utiliser en pressant manuellement le réservoir.

6. Dispositif selon l'une quelconque des revendications précédentes, dans lequel la pointe (112) est associée à une marque hydrochromique pour indiquer la quantité de matière aqueuse prélevée.

7. Dispositif selon l'une quelconque des revendications précédentes, dans lequel le réservoir (128) contient une formule de réactif liquide pour le traitement, de préférence l'élution, de l'échantillon obtenu sur la pointe (112).

8. Dispositif selon l'une quelconque des revendications précédentes, dans lequel la pointe (112) comprend un agent actif pour le traitement de l'échantillon obtenu sur la pointe, et de préférence la pointe est fonctionnalisée avec ledit agent actif, de préférence dans lequel l'agent actif comprend un ou plusieurs réactifs perturbant la membrane qui ont la capacité de lyser des bactéries ou virus.

9. Dispositif selon l'une quelconque des revendications précédentes, comprenant en outre une chambre de réaction (114) pour recevoir le fluide fourni au départ du réservoir (128) et évacué au départ de la pointe (112) après contact avec l'échantillon, de préférence dans lequel la chambre de réaction (114) peut être placée de façon amovible de manière à couvrir la pointe (112).

10. Dispositif selon l'une quelconque des revendications précédentes, comprenant une formule de réactif (150) pour le traitement de l'échantillon, de préférence dans lequel la formule de réactif (150) est sous une forme lyophilisée ou séchée.

11. Dispositif selon la revendication 10, dans lequel la formule de réactif (150) comprend un ou plusieurs réactifs pour la détection de cibles d'acide nucléique spécifiques dans l'échantillon, de préférence dans lequel ladite formule de réactif (150) comprend des réactifs pour l'amplification isotherme de l'acide nucléique, et de façon particulièrement préférée dans lequel l'amplification isotherme est une amplification par recombinase polymérase.

12. Dispositif selon l'une quelconque des revendications 10 ou 11, dans lequel la formule de réactif (150) fournit un signal visuel en cas de détection desdites cibles d'acide nucléique spécifiques.

13. Dispositif selon la revendication 10, dans lequel la formule de réactif (150) comprend un ou plusieurs réactif(s) pour la détection de protéines, peptides et/ou lipides spécifiques dans l'échantillon, de préférence dans lequel la formule de réactif (150) comprend un ou plusieurs réactifs pour la détection par immunoessai d'une cible dans l'échantillon.

14. Dispositif selon l'une quelconque des revendications précédentes, fourni sous forme de kit.

15. Utilisation d'un dispositif selon l'une quelconque des revendications précédentes pour la collecte et la préparation d'un échantillon viral destiné à être utilisé dans un test à flux latéral, de préférence dans lequel l'échantillon viral est un échantillon de SARS-CoV-2.
